(19) Europäisches Patentamt
European Patent Office
Office européen des brevets

(11) Veröffentlichungsnummer: **0 130 518**
**A1**

(12) # EUROPÄISCHE PATENTANMELDUNG

(21) Anmeldenummer: **84107252.3**

(22) Anmeldetag: **25.06.84**

(51) Int. Cl.⁴: **A 01 N 43/64**, C 07 D 253/06

(30) Priorität: **02.07.83 DE 3323933**

(43) Veröffentlichungstag der Anmeldung: **09.01.85**
**Patentblatt 85/2**

(84) Benannte Vertragsstaaten: **BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Kranz, Eckart, Dr., Am Acker 9, D-5600 Wuppertal 1 (DE)**
Erfinder: **Findeisen, Kurt, Dr., In der Follmühle 10, D-5068 Odenthal 1 (DE)**
Erfinder: **Eue, Ludwig, Dr., Paul-Klee-Strasse 36, D-5090 Leverkusen 1 (DE)**
Erfinder: **Schmidt, Robert. R., Dr., Im Waldwinkel 110, D-5060 Bergisch-Gladbach 2 (DE)**

(54) **6-Cyclobutyl-1,2,4-triazin-5-on-Derivate.**

(57) Neue 6-Cyclobutyl-1,2,4-triazin-5-on-Derivate der allgemeinen Formel (I),

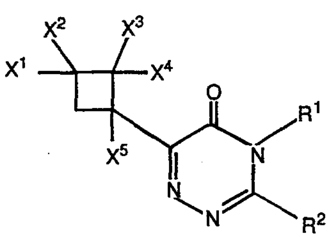

(I)

in welcher
$R^1$ für Amino oder die Gruppierung $-N=CR^3R^4$ steht,
$R^2$ für Alkylthio, Alkylamino, Dialkylamino oder Alkenylamino steht,
$R^3$ für Wasserstoff oder Alkyl steht,
$R^4$ für Alkyl, Cycloalkyl, Cycloalkenyl oder gegebenenfalls substituiertes Phenyl steht,
$X^1$, $X^2$, $X^3$ und $X^4$ gleich oder verschieden sind und für Wasserstoff oder Halogen stehen und
$X^5$ für Wasserstoff oder Alkyl steht,

ein Verfahren zu ihrer Herstellung und ihre Verwendung als Herbizide.

EP 0 130 518 A1

0130518

BAYER AKTIENGESELLSCHAFT 5090 Leverkusen, Bayerwerk

**Konzernverwaltung** RP
**Patentabteilung**

Bi/AB
Ia

# 6-Cyclobutyl-1,2,4-triazin-5-on-Derivate

Die vorliegende Erfindung betrifft neue 6-Cyclobutyl-1,2,4-triazin-5-on-Derivate, ein Verfahren zu ihrer Herstellung sowie ihre Verwendung als Herbizide, insbesondere als selektive Herbizide.

Es ist bereits bekannt geworden, daß bestimmte substituierte 1,2,4-Triazin-5-on-Derivate, wie beispielsweise 6-Chlor(Fluor)-tert.-butyl-4-isopropylidenamino-3-methylthio-1,2,4-triazin-5-on, als Herbizide verwendet werden können (vgl. EP 0 074 539 [LeA 21 174]). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

Es wurden neue 6-Cyclobutyl-1,2,4-triazin-5-on-Derivate der allgemeinen Formel (I),

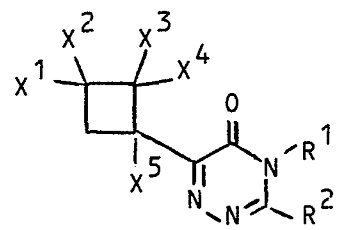

(I)

in welcher

$R^1$ für Amino oder die Gruppierung $-N=CR^3R^4$ steht,

$R^2$ für Alkylthio, Alkylamino, Dialkylamino oder Alkenylamino steht,

$R^3$ für Wasserstoff oder Alkyl steht,

$R^4$ für Alkyl, Cycloalkyl, Cycloalkenyl oder gegebenenfalls substituiertes Phenyl steht,

$X^1$, $X^2$, $X^3$ und $X^4$ gleich oder verschieden sind und für Wasserstoff oder Halogen stehen und

$X^5$ für Wasserstoff oder Alkyl steht,

aufgefunden.

Weiterhin wurde gefunden, daß man die 6-Cyclobutyl-1,2,4-triazin-5-on-Derivate der Formel (I) erhält, wenn man in einer <u>ersten Stufe</u> Cyclobutancarbonsäurecyanide

0130518

der Formel (II),

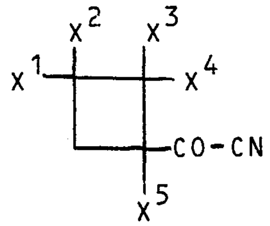

(II)

in welcher

$X^1, X^2, X^3, X^4$ und $X^5$ die oben angegebene Bedeutung haben,

a) gegebenenfalls in Gegenwart einer flüssigen Carbonsäure als Lösungsmittel mit einer anorganischen Säure umsetzt oder

b) an ein Carboniumion addiert, das aus einem Olefin, wie z.B. Isobutylen, oder einem tertiären Alkohol, wie z.B. tert.-Butanol, mit einer starken Säure, wie insbesondere Schwefelsäure, gebildet wird und anschließend der Hydrolyse unterwirft (sog. RITTERREAKTION).

und

die hierbei entstehenden Cyclobutanketocarbonsäureamide der Formel (IIIa),

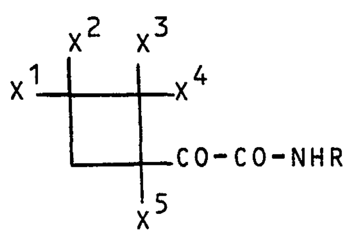

(IIIa)

in welcher

$X^1, X^2, X^3, X^4$ und $X^5$ die oben angegebene Bedeutung haben und

R für Wasserstoff (erhältlich nach Variante a) oder Alkyl, insbesondere tert.-Butyl (erhältlich nach Variante b) steht,

in einer <u>zweiten Stufe</u> in an sich bekannter Weise entweder direkt in der anfallenden Lösung oder nach Zwischenisolierung, gegebenenfalls nach vorheriger Verseifung zu den freien Cyclobutylketocarbonsäuren der Formel (IIIb),

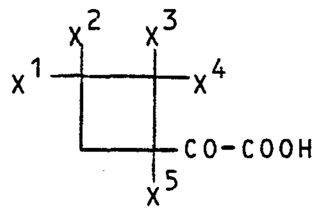

(IIIb)

in welcher

$X^1, X^2, X^3, X^4$ und $X^5$ die oben angegebene Bedeutung haben,

mit Thiocarbohydrazid der Formel (IV)

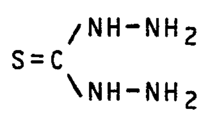

(IV)

in wässriger bzw. in wässrig-saurer Lösung, gegebenenfalls in Gegenwart eines organischen Verdünnungsmittels, zu 4-Amino-6-cyclobutyl-3-mercapto-1,2,4-triazin-5-onen der Formel (V),

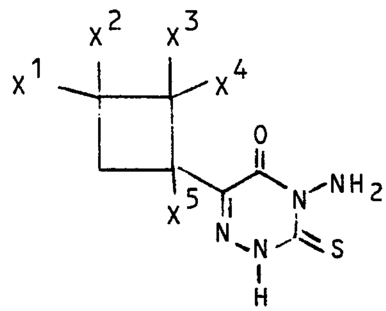

(V)

in welcher

$X^1, X^2, X^3, X^4$ und $X^5$ die oben angegebene Bedeutung haben,

umsetzt; diese in einer dritten Stufe in bekannter Weise in alkoholischer Lösung mittels Alkylhalogenid, vorzugsweise Alkyljodid oder -bromid, zu 3-Alkylthio-4-amino-6-cyclobutyl-1,2,4-triazin-5-onen der Formel (Ia),

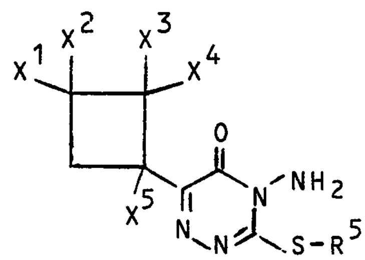

(Ia)

in welcher

$X^1, X^2, X^3, X^4$ und $X^5$ die oben angegebene Bedeutung haben und

$R^5$ für Alkyl, vorzugsweise mit 1 bis 4 Kohlenstoffatomen, steht,

alkyliert, diese gegebenenfalls in einer vierten Stufe mit Aminen der Formel (VI),

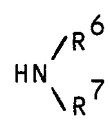

(VI)

in welcher

$R^6$ für Wasserstoff oder Alkyl, vorzugsweise mit 1 bis 6 Kohlenstoffatomen, steht und

$R^7$ für Alkyl, vorzugsweise mit 1 bis 6 Kohlenstoffatomen, oder Alkenyl, vorzugsweise mit 3 bis 6 Kohlenstoffatomen, steht,

in Gegenwart eines Verdünnungsmittels umsetzt und gegebenenfalls in einer <u>fünften Stufe</u> die gemäß der dritten bzw. vierten Stufe entstandenen 4-Amino-6-cyclobutyl-1,2,4-triazin-5-one der Formel (Ib),

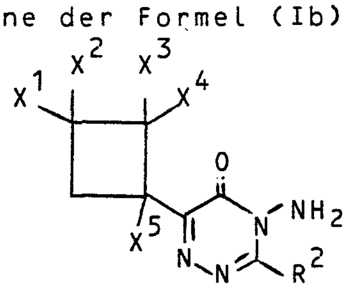

(Ib)

in welcher

$R^2, X^1, X^2, X^3, X^4$ und $X^5$ die oben angegebene Bedeutung haben,

mit Carbonylverbindungen der Formel (VII),

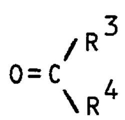

(VII)

in welcher

$R^3$ und $R^4$ die oben angegebene Bedeutung haben,

gegebenenfalls in Gegenwart eines Verdünnungsmittels und gegebenenfalls in Gegenwart eines basischen oder sauren Katalysators umsetzt; die Carbonylverbindungen der Formel (VII) können dabei gegebenenfalls in Form von Acetalen bzw. Ketalen eingesetzt werden.

Außerdem wurde gefunden, daß die 6-Cyclobutyl-1,2,4-triazin-5-on-Derivate der Formel (I) gute herbizide, insbesondere selektiv-herbizide Eigenschaften aufweisen.

Ueberraschenderweise zeigen die erfindungsgemäßen Verbindungen gegenüber den bekannten 6-Chlor(Fluor)-tert.-butyl-4-isopropylidenamino-3-methylthio-1,2,4-triazin-5-onen, welches chemisch und strukturell naheliegende Verbindungen sind, neben einer besseren allgemeinen herbiziden Wirkung, insbesondere eine bessere Verträglichkeit bei wichtigen Kulturpflanzen, wie bei Mais, Weizen und anderen. Die erfindungsgemäßen Wirkstoffe stellen somit eine Bereicherung der herbiziden Mittel, insbesondere der selektiven chemischen Unkrautbekämpfung dar.

Die erfindungsgemäßen 6-Cyclobutyl-1,2,4-triazin-5-on-Derivate sind durch die Formel (I) allgemein definiert. In dieser Formel stehen vorzugsweise

$R^1$ für Amino oder die Gruppierung $-N=CR^3R^4$,

$R^2$ für geradkettiges oder verzweigtes Alkylthio mit 1 bis 4 Kohlenstoffatomen, für Alkylamino und Dialkylamino mit jeweils 1 bis 6 Kohlenstoffatomen in jedem Alkylteil sowie für Alkenylamino mit 3 bis 6 Kohlenstoffatomen,

$R^3$ für Wasserstoff oder geradkettiges oder verzweigtes Alkyl mit 1 bis 6 Kohlenstoffatomen,

$R^4$ für geradkettiges oder verzweigtes Alkyl mit 1 bis 6 Kohlenstoffatomen, für Cycloalkyl mit 3 bis 6 Kohlenstoffatomen oder Cycloalkenyl mit 5 oder 6 Kohlenstoffatomen, für gegebenenfalls substituiertes Phenyl, wobei als Substituenten beispielsweise genannt seien :

Halogen, Alkyl mit 1 bis 4 Kohlenstoffatomen, Halogenalkyl mit 1 bis 2 Kohlenstoffatomen und bis zu 5 gleichen oder verschiedenen Halogenatomen (wie vorzugsweise Fluor- und Chloratomen), Alkoxy und Alkylthio mit jeweils bis 2 Kohlenstoffatomen, Cyano und Nitro,

$X^1$, $X^2$, $X^3$ und $X^4$ für Wasserstoff oder Halogen, wobei diese Substituenten gleich oder verschieden sein können und

$X^5$ für Wasserstoff oder Alkyl mit 1 bis 4 Kohlenstoffatomen.

Besonders bevorzugt sind diejenigen Verbindungen der Formel (I), in denen

$R^1$ die oben angegebene Bedeutung hat,

$R^2$ für Methyl-, Ethyl- oder Propylthio, ferner für Methyl-, Ethyl-, Propyl- oder Hexylamino und für Dimethyl-, Diethyl- oder Ethylmethylamino sowie für Allylamino steht,

$R^3$ für Wasserstoff oder geradkettiges oder verzweigtes Alkyl mit 1 bis 4 Kohlenstoffatomen steht,

$R^4$ für geradkettiges oder verzweigtes Alkyl mit 1 bis 4 Kohlenstoffatomen, Cyclohexyl, Cyclohexenyl oder für gegebenenfalls substituiertes Phenyl steht, wobei als Substituenten genannt seien :

Fluor, Chlor, Methyl, Ethyl und Nitro,

$X^1$, $X^2$, $X^3$ und $X^4$ gleich oder verschieden sind und für Wasserstoff, Fluor oder Chlor stehen und

$X^5$ für Wasserstoff, Methyl oder Ethyl steht.

Verwendet man beispielsweise Cyclobutancarbonsäurecyanid als Ausgangsstoff in der ersten Stufe, setzt in der zweiten Stufe die entsprechende freie Säure mit Thiocarbohydrazid um und verwendet in der dritten Stufe Methylbromid als Alkylierungsmittel, so kann der Reaktionsablauf durch das folgende Formelschema wiedergegeben werden :

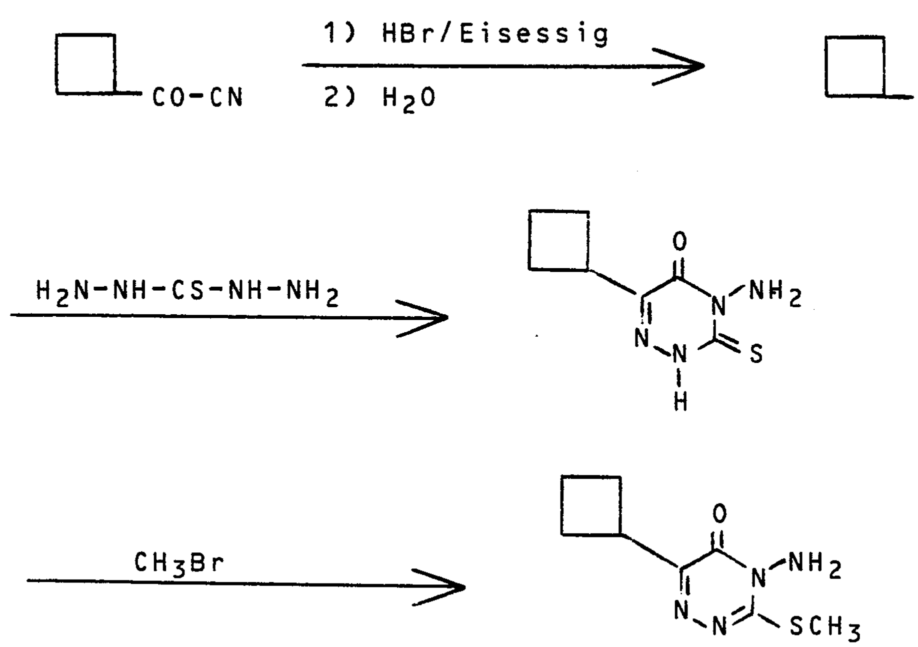


Verwendet man das so erhaltene 4-Amino-6-cyclobutyl-3-methylthio-1,2,4-triazin-5-on und Dimethylamin als Ausgangsstoffe für die vierte Stufe, so kann der Reaktionsablauf durch das folgende Formelschema wiedergegeben werden :

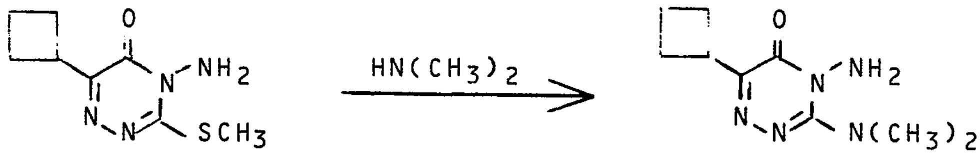


Verwendet man beispielsweise 4-Amino-6-cyclobutyl-3-methylthio-1,2,4-triazin-5-on und Aceton als Ausgangsstoffe für die fünfte Stufe, so kann der Reaktionsablauf durch das folgende Formelschema wiedergegeben werden :

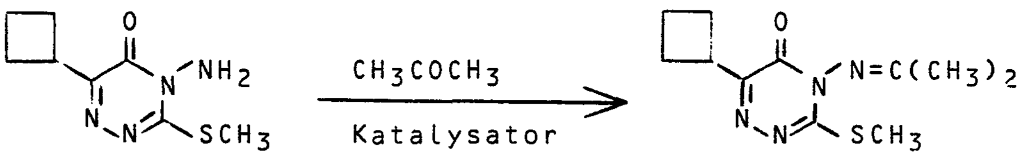


Die für das erfindungsgemäße Verfahren als Ausgangsstoffe zu verwendenden Cyclobutancarbonsäurecyanide sind durch die Formel (II) allgemein definiert. In dieser Formel stehen $X^1$, $X^2$, $X^3$, $X^4$ und $X^5$ vorzugsweise für die Reste, die bereits im Zusammenhang mit der Beschreibung der erfindungsgemäßen Stoffe der Formel (I) vorzugsweise für diese Substituenten genannt wurden.

Die Cyclobutancarbonsäurecyanide der Formel (II) sind bekannt (vgl. z.B. Monatsh. Chem. 96, 1983-1989 (1965)); bzw. können sie in bekannter Art und Weise erhalten werden, indem man entsprechende Halogenide mit Trimethylsilylcyanid gegebenenfalls in Gegenwart eines Verdünnungsmittels umsetzt. Trimethylsilylcyanid, $(CH_3)_3Si-CN$, ist bekannt (vgl. z.B. Synthesis 1979, S. 522 und 523).

Bekannt sind die Amine der Formel (VI), die Carbonylverbindungen der Formel (VII), d.h. Aldehyde und Ketone, sowie das Thiocarbohydrazid der Formel (IV).

Die erste Stufe des erfindungsgemäßen Verfahrens kann in Abwesenheit oder in Gegenwart einer unter den Reaktionsbedingungen flüssigen, niederaliphatischen Carbonsäure als Lösungsmittel durchgeführt werden. Als solche Lösungsmittel kommen vorzugsweise Carbonsäuren mit 1 bis 6 Kohlenstoffatomen, wie Essigsäure, Propionsäure oder Ameisensäure, infrage.

Die erste Stufe des erfindungsgemäßen Verfahrens wird mit Hilfe einer starken anorganischen Säure durchgeführt. Hierzu gehören vorzugsweise die Halogenwasserstoffsäuren, wie Chlorwasserstoff und Bromwasserstoff, sowie konzentrierte Schwefelsäure.

Die Reaktionstemperaturen können bei der Durchführung dieser Verfahrensstufe in einem größeren Bereich variiert werden. Im allgemeinen arbeitet man zwischen -20 und +50°C, vorzugsweise zwischen 0 und +40°C.

Bei der Durchführung der ersten Stufe des erfindungsgemäßen Verfahrens setzt man auf 1 Mol Cyclobutancarbonsäurecyanid der Formel (II) vorzugsweise 1 bis 10 Mol an anorganischer Säure ein.

Die zweite Stufe des erfindungsgemäßen Verfahrens erfolgt vorzugsweise ohne vorherige Zwischenisolierung der Cyclobutanketocarbonsäureamide der Formel (IIIa) und nach Verseifung derselben in üblicher Weise zu freien Säuren der Formel (IIIb).

Die zweite Stufe des erfindungsgemäßen Verfahrens wird in wäßriger Lösung bzw. in Gegenwart einer wäßrig-sauren Lösung wie einer halogenwassserstoffsauren, vorzugsweise einer salzsauren oder einer schwefelsauren Lösung durchgeführt.

Wird in Gegenwart eines organischen Lösungsmittels gearbeitet, so kommen alle üblichen organischen Lösungsmittel in Frage, wie insbesondere Dimethylformamid.

Die Reaktionstemperaturen können bei der Durchführung der zweiten Stufe des erfindungsgemäßen Verfahrens in einem größeren Bereich variiiert werden. Im allgemeinen arbeitet man zwischen 0 und 120°C, vorzugsweise zwischen 0 und 100°C.

Bei der Durchführung der zweiten Stufe des erfindungsgemäßen Verfahrens setzt man die Ausgangsstoffe vorzugsweise in molaren Mengen bzw. mit einem Überschuß an Thiocarbohydrazid der Formel (IV) ein. Die Isolierung der Zwischenprodukte der Formel (V) erfolgt in üblicher Weise.

Die dritte Stufe des erfindungsgemäßen Verfahrens wird in Gegenwart einer Base durchgeführt. Hierbei werden vorzugsweise Alkalihydroxide, wie Natriumhydroxid, in wäßriger Lösung oder Alkalialkoholate, wie Natriummethylat, wobei überschüssiger Alkohol als Lösungsmittel verwendet wird, eingesetzt.

Die Reaktionstemperaturen können bei der Durchführung der dritten Stufe des erfindungsgemäßen Verfahrens in einem größeren Bereich variiert werden. Im allgemeinen arbeitet man zwischen 0 und 100°C, vorzugsweise zwischen 0 und 50°C.

Bei der Durchführung der dritten Stufe des erfindungsgemäßen Verfahrens setzt man auf 1 Mol Zwischenprodukt der Formel (V) vorzugsweise 1 bis 1,5 Mol Alkylierungsmittel ein. Die Isolierung der Zwischenprodukte bzw. Endprodukte der Formel (Ia) erfolgt in üblicher Weise.

Als Verdünnungsmittel kommen für die vierte Stufe des erfindungsgemäßen Verfahrens vorzugsweise organische Lösungsmittel in Frage, wie insbesondere Isopropanol/Eisessig.

Die Reaktionstemperaturen können bei der Durchführung der vierten Stufe des erfindungsgemäßen Verfahrens in einem größeren Bereich variiert werden. Im allgemeinen arbeitet man zwischen 20 und 180°C, vorzugsweise zwischen 40 und 150°C.

Bei der Durchführung der vierten Stufe des erfindungsgemäßen Verfahrens setzt man auf 1 Mol der Verbindung der Formel (Ia) vorzugsweise 1 bis 3 Mol Amin der Formel (VI) ein. Die Isolierung der Endprodukte erfolgt in üblicher Weise.

Als Verdünnungsmittel kommen für die fünfte Stufe des erfindungsgemäßen Verfahrens alle inerten organischen Lösungsmittel in Frage. Hierzu gehören vorzugsweise Alkohole, wie Methanol, Ethanol und tert.-Butanol; Ether, wie insbesondere Tetrahydrofuran und Dioxan; halogenierte Kohlenwasserstoffe, wie Methylenchlorid; sowie aromatische Kohlenwasserstoffe, wie Benzol, Toluol oder Xylol.

0130518

Die fünfte Stufe des erfindungsgemäßen Verfahrens wird gegebenenfalls in Gegenwart eines basischen oder eines sauren Katalysators durchgeführt. Hierbei können alle üblichen basischen Katalysatoren eingesetzt werden, wie z.B. Dimethylbenzylamin. Als saure Katalysatoren kommen insbesondere p-Toluolsulfonsäure oder saure Kationenaustauscher in Betracht.

Die Reaktionstemperaturen können bei der Durchführung der fünften Stufe des erfindungsgemäßen Verfahrens in einem größeren Bereich variiert werden. Im allgemeinen arbeitet man bei Temperaturen zwischen -50 und +150°C, vorzugsweise zwischen -10 und + 120°C.

Bei der Durchführung der fünften Stufe des erfindungsgemäßen Verfahrens setzt man vorzugsweise auf 1 Mol 4-Amino-6-cyclobutyl-1,2,4-triazin-5-on der Formel (Ib) 1 bis 2 Mol einer Carbonylverbindung der Formel (VII) und gegebenenfalls 0,01 bis 0,1 Mol Katalysator ein. Die Isolierung der Verbindungen der Formel (I) erfolgt in üblicher Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbiziden Wirkung eine gute Verträglichkeit gegenüber Nutzpflanzen. So ist es möglich, wichtige Schadgräser selektiv in wichtigen Kulturpflanzen, wie z.B. in Mais und Baumwolle zu bekämpfen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,05 und 10 kg Wirkstoff pro Hektar Bodenfläche, vorzugsweise zwischen 0,1 und 5 kg pro ha.

Die nachfolgenden Beispiele dienen zur weiteren Erläuterung der Erfindung.

0130518

Herstellungsbeispiele :

Beispiel 1 :

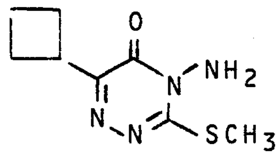

(1)

(1. und 2. Stufe )

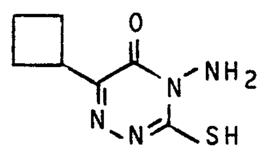

17,5 g (0,16 Mol) Cyclobutancarbonsäurecyanid werden bei Raumtemperatur zu 128,3 g Bromwasserstoff in Eisessig gegeben. Man läßt 2 Stunden bei Raumtemperatur nachrühren und versetzt dann unter Kühlung mit 2,9 ml Wasser, so daß die Innentemperatur 24°C nicht übersteigt. Man läßt wiederum 2 Stunden bei Raumtemperatur nachrühren und gibt dann 18,7 g (0,176 Mol) Thiocarbohydrazid in 180 ml 1n Salzsäure zu. Man läßt über Nacht bei Raumtemperatur nachrühren, saugt den ausgefallenen Niederschlag ab, wäscht mit Wasser nach und trocknet bei 50°C im Vakuum. Man erhält 21 g (66 % der Theorie) 4-Amino-6-cyclobutyl-3-mercapto-1,2,4-triazin-5-on vom Schmelzpunkt 187-194°C.

(3. Stufe)

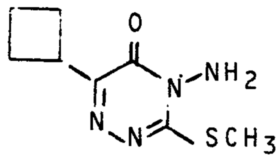

(1)

19,8 g (0,1 Mol) 4-Amino-6-cyclobutyl-3-mercapto-1,2,4-triazin-5-on (gemäß Stufe 2) werden in 100 ml 1n Natronlauge gelöst. Nach vollständiger Lösung werden bei Raumtemperatur 17,7 g (0,125 Mol) Methyljodid zugetropft. Nach beendeter Zugabe läßt man über Nacht bei Raumtemperatur nachrühren. Danach wird der entstehende Feststoff abgesaugt, mit Wasser gewaschen, getrocknet und in Essigester mit Aktivkohle umkristallisiert. Man erhält 13,3 g (63 % der Theorie) 4-Amino-6-cyclobutyl-3-methylthio-1,2,4-triazin-5-on vom Schmelzpunkt 118-120°C.

Herstellung des Ausgangsproduktes:

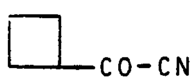

107 g (0,86 Mol) Cyclobutancarbonsäurechlorid werden auf 110 bis 120°C erwärmt. Innerhalb von 30 Minuten tropft man 89 g (0,9 Mol) Trimethylsilylcyanid zu. Gleichzeitig wird das entstehende Trimethylsilylchlorid destillativ aus der Reaktionsmischung entfernt.

Man läßt 30 Minuten bei 120°C nachrühren und destilliert das Reaktionsprodukt. Man erhält 39 g Cyclobutancarbonsäurecyanid vom Siedepunkt 53-57°C/18 mbar.

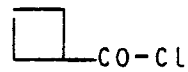

Zu 373,1 g (3,73 Mol) Cyclobutancarbonsäure werden bei Raumtemperatur langsam 487,8 g (4,1 Mol) Thionylchlorid getropft. Man erwärmt die Reaktionslösung langsam auf 70°C und läßt bis zur Beendigung der Gasentwicklung nachrühren. Die Reaktionslösung wird fraktioniert. Man erhält 381,6 g (86 % der Theorie) Cyclobutancarbonsäurechlorid vom Siedepunkt 91-123°C.

<u>Beispiel 2 :</u>

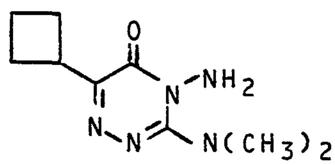

(2)

In eine Lösung von 4,8 g (0,08 Mol) Eisessig in 130 ml Isopropanol werden bei 5°C unter starker Kühlung ca. 0,15 Mol Dimethylamin eingeleitet. Diese Reaktionslösung wird anschließend mit 8,5 g (0,04 Mol) 4-Amino-6-cyclobutyl-3-methylthio-1,2,4-triazin-5-on (Herstellung gemäß Beispiel 1) versetzt. Man läßt auf Raumtemperatur erwärmen und erhitzt anschließend 24 Stunden unter Rückfluß. Danach wird das Reaktionsgemisch eingeengt. Der Rückstand wird in Methylenchlorid aufgenommen und mit 1n Salzsäure extrahiert. Die Salzsäurephase wird auf pH 14 gestellt, mit Methylenchlorid extrahiert, über Natriumsulfat getrocknet und eingeengt. Der ölige Rückstand

wird in n-Hexan zur Kristallisation gebracht. Man erhält 6,2 g (74 % der Theorie) 4-Amino-6-cyclobutyl-3-dimethylamino-1,2,4-triazin-5-on vom Schmelzpunkt 90-91°C.

Beispiel 3 :

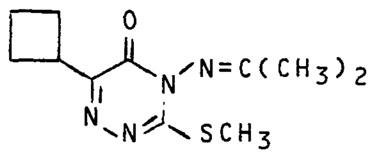

(3)

8,5 g (0,04 Mol) 4-Amino-6-cyclobutyl-3-methylthio-1,2,4-triazin-5-on (Herstellung gemäß Beispiel 1) werden in 600 ml Aceton aufgeschlämmt und bei Raumtemperatur mit 1 ml Bromwasserstoff in Eisessig versetzt. Die Reaktionslösung wird über Nacht bei Raumtemperatur gerührt. Danach engt man ein, nimmt den Rückstand in Methylenchlorid auf, wäscht mit Wasser, trocknet über Natriumsulfat und engt erneut ein. Nach Trennung über eine Kieselgelsäule (Chloroform/Ether : 8/2) erhält man 2,3 g (22,8 % der Theorie) 6-Cyclobutyl-4-isopropylidenamino-3-methylthio-1,2,4-triazin-5-on vom Schmelzpunkt 75-76°C.

In analoger Weise und entsprechend dem erfindungsgemäßen Verfahren werden die in der nachfolgenden Tabelle aufgeführten Verbindungen der Formel (I) erhalten :

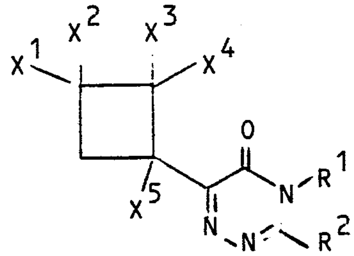

(I)

Tabelle 1:

| Beisp.-Nr. | 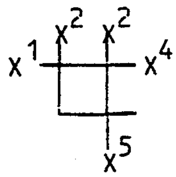 | $R^1$ | $R^2$ | Schmelzpunkt(°C) bzw. $n_D^{20}$ |
|---|---|---|---|---|
| 4 | 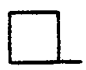 | $-NH_2$ | $-NHCH_3$ | 162-163 |
| 5 | 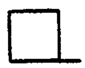 | $-NH_2$ | $-N{<}^{CH_3}_{C_2H_5}$ | 1,570 |
| 6 | 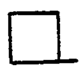 | $-NH_2$ | $-NH-CH_2-CH=CH_2$ | 142-143 |
| 7 | 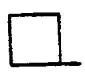 | $-N=C{<}^{CH_3}_{CH_3}$ | $-NHCH_3$ | 133-135 |
| 8 | 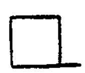 | $-NH_2$ | $-SC_2H_5$ | 140-141 |
| 9 | 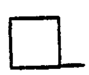 | $-N=C{<}^{CH_3}_{CH_3}$ | $-SC_2H_5$ | 85-87 |
| 10 | 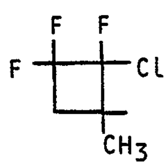 | $-NH_2$ | $-SCH_3$ | 194-195 |
| 11 | 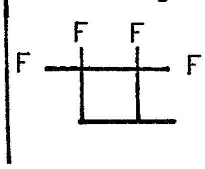 | $-NH_2$ | $-SCH_3$ | 165-168 |

Verwendungsbeispiele :

In den nachfolgenden Beispielen werden die nachstehend angegebenen Verbindungen als Vergleichssubstanzen eingesetzt :

(A) 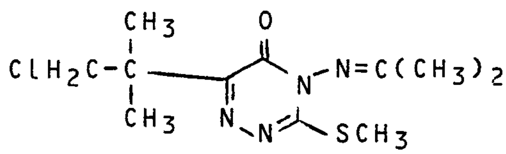

6-Chlor-tert.-butyl-4-isopropylidenamino-3-methylthio-1,2,4-triazin-5-on

(B) 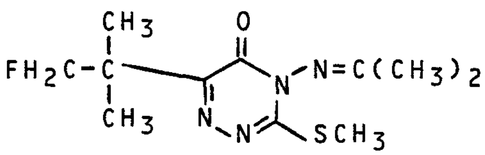

6-Fluor-tert.-butyl-4-isopropylidenamino-3-methylthio-1,2,4-triazin-5-on

beide bekannt aus EP-A-0 074 539 (= DE-OS 31 35 413).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigt z.B. die Verbindung gemäß dem Herstellungsbeispiel (3) neben einer besseren allgemeinen herbiziden Wirksamkeit insbesondere eine bessere Selektivität in Weizen als die aus dem Stand der Technik bekannte Verbindung (B).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die Verbindung gemäß dem Herstellungsbeispiel (5) neben einer besseren allgemeinen herbiziden Wirksamkeit insbesondere eine bessere Selektivität in Baumwolle, Hafer, Weizen und Mais als die aus dem Stand der Technik bekannte Verbindung (A).

Patentansprüche

1. 6-Cyclobutyl-1,2,4-triazin-5-on-Derivate der allgemeinen Formel (I),

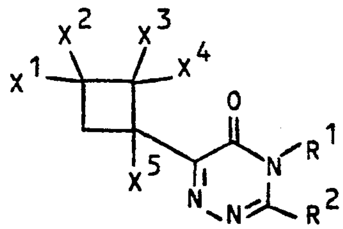

(I)

in welcher

$R^1$ für Amino oder die Gruppierung $-N=CR^3R^4$ steht,

$R^2$ für Alkylthio, Alkylamino, Dialkylamino oder Alkenylamino steht,

$R^3$ für Wasserstoff oder Alkyl steht,

$R^4$ für Alkyl, Cycloalkyl, Cycloalkenyl oder gegebenenfalls substituiertes Phenyl steht,

$X^1$, $X^2$, $X^3$ und $X^4$ gleich oder verschieden sind und für Wasserstoff oder Halogen stehen und

$X^5$ für Wasserstoff oder Alkyl steht.

2. 6-Cyclobutyl-4-isopropylidenamino-3-methylthio-1,2,4-triazin-5-on der Formel

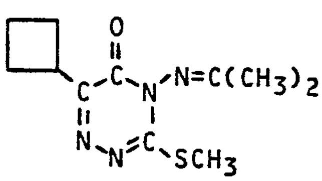

(3)

gemäß Anspruch 1.

3. 6-Cyclobutyl-3-(N-methyl-N-ethyl-amino)-4-amino-1,2,4-triazin-5-on der Formel

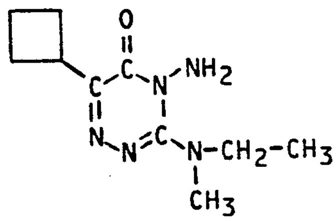

(5)

gemäß Anspruch 1.

4. Verfahren zur Herstellung von 6-Cyclobutyl-1,2,4-triazin-5-on-Derivaten der allgemeinen Formel (I) gemäß Anspruch 1, dadurch gekennzeichnet, daß man in einer <u>ersten Stufe</u> Cyclobutancarbonsäurecyanide der Formel (II),

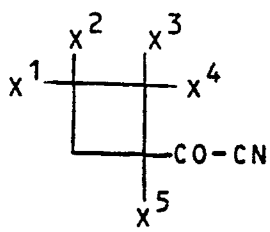

(II)

in welcher
$X^1$, $X^2$, $X^3$, $X^4$ und $X^5$ die oben angegebene Bedeutung haben,

(a) gegebenenfalls in Gegenwart einer flüssigen Carbonsäure als Lösungsmittel mit einer anorganischen Säure umsetzt oder

(b) an ein Carboniumion addiert, das aus einem Olefin oder einem tertiären Alkohol mit einer starken Säure gebildet wird, und anschließend der Hydrolyse unterwirft (sog. RITTERREAKTION)

und

die hierbei entstehenden Cyclobutanketocarbonsäureamide der Formel (IIIa),

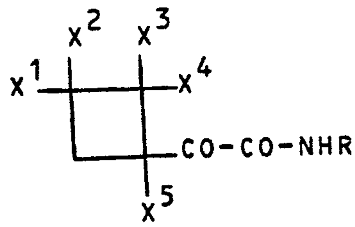

(IIIa)

in welcher

$X^1, X^2, X^3, X^4$ und $X^5$ die oben angegebene Bedeutung haben und

R für Wasserstoff (erhältlich nach Variante a) oder Alkyl, insbesondere tert.-Butyl (erhältlich nach Variante b) steht,

in einer <u>zweiten Stufe</u> in an sich bekannter Weise entweder direkt in der anfallenden Lösung oder nach Zwischenisolierung, gegebenenfalls nach vorheriger Verseifung zu den freien Cyclobutylketocarbonsäuren der Formel (IIIb),

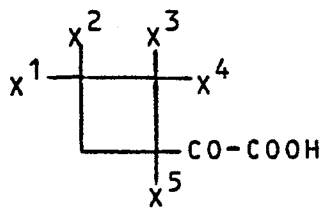

(IIIb)

in welcher

$X^1, X^2, X^3, X^4$ und $X^5$ die oben angegebene Bedeutung haben,

mit Thiocarbohydrazid der Formel (IV)

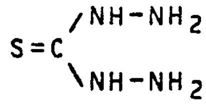

(IV)

in wässriger bzw. in wässrig-saurer Lösung, gegebenenfalls in Gegenwart eines organischen Verdünnungsmittels, zu 4-Amino-6-cyclobutyl-3-mercapto-1,2,4-triazin-5-onen der Formel (V),

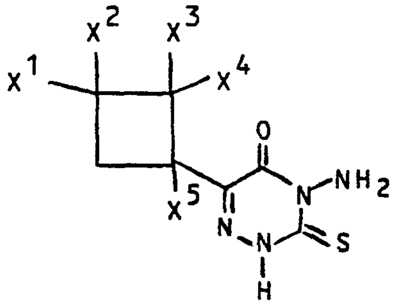

(V)

in welcher

$X^1, X^2, X^3, X^4$ und $X^5$ die oben angegebene Bedeutung haben,

umsetzt; diese in einer <u>dritten Stufe</u> in bekannter Weise in alkoholischer Lösung mittels Alkylhalogenid, vorzugsweise Alkyljodid oder -bromid, zu 3-Alkylthio-4-amino-6-cyclobutyl-1,2,4-triazin-5-onen der Formel (Ia),

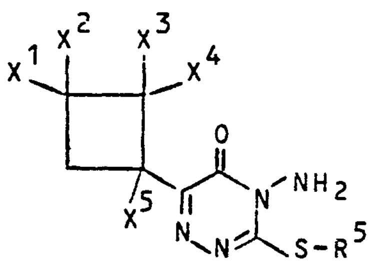

(Ia)

in welcher

$X^1, X^2, X^3, X^4$ und $X^5$ die oben angegebene Bedeutung haben

und

$R^5$ für Alkyl, vorzugsweise mit 1 bis 4 Kohlenstoffatomen steht,

alkyliert, diese gegebenenfalls in einer <u>vierten Stufe</u> mit Aminen der Formel (VI),

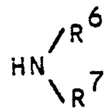

(VI)

in welcher

$R^6$ für Wasserstoff oder Alkyl, vorzugsweise mit 1 bis 6 Kohlenstoffatomen, steht und

$R^7$ für Alkyl, vorzugsweise mit 1 bis 6 Kohlenstoffatomen, oder Alkenyl, vorzugsweise mit 3 bis 6 Kohlenstoffatomen, steht,

in Gegenwart eines Verdünnungsmittels umsetzt und gegebenenfalls in einer <u>fünften Stufe</u> die gemäß der dritten bzw. vierten Stufe entstandenen 4-Amino-6-cyclobutyl-1,2,4-triazin-5-one der Formel (Ib),

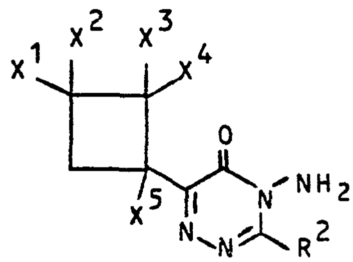

(Ib)

in welcher

$R^2, X^1, X^2, X^3, X^4$ und $X^5$ die oben angegebene Bedeutung haben,

mit Carbonylverbindungen der Formel (VII),

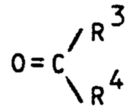 (VII)

in welcher

$R^3$ und $R^4$ die oben angegebene Bedeutung haben,

gegebenenfalls in Gegenwart eines Verdünnungsmittels und gegebenenfalls in Gegenwart eines basischen oder sauren Katalysators umsetzt, wobei die Carbonylverbindungen der Formel (VII) gegebenenfalls in Form von Acetalen bzw. Ketalen eingesetzt werden können.

5. Herbizide Mittel, gekennzeichnet durch einen Gehalt an mindestens einem 6-Cyclobutyl-1,2,4-triazin-5-on-Derivat der Formel (I) gemäß Anspruch 1.

6. Verwendung von 6-Cyclobutyl-1,2,4-triazin-5-on-Derivaten der Formel (I) gemäß Anspruch 1 zur Bekämpfung von unerwünschtem Pflanzenwachstum.

7. Verfahren zur Herstellung von herbiziden Mitteln, dadurch gekennzeichnet, daß man 6-Cyclobutyl-1,2,4-triazin-5-on-Derivate der Formel (I) gemäß Anspruch 1 mit Streckmitteln und/oder oberflächenaktiven Mitteln vermischt.

Europäisches Patentamt

# EUROPÄISCHER RECHERCHENBERICHT

0130518
Nummer der Anmeldung

EP 84 10 7252

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | EP-A-0 044 696 (SUMITOMO CHEMICAL COMPANY, LTD.) * Beispiele 81,82 * | 1,5 | A 01 N 43/64<br>C 07 D 253/06 |
| D,Y | EP-A-0 074 539 (BAYER AG) * Ansprüche * | 1,5 | |
| Y | EP-A-0 074 538 (BAYER AG) * Ansprüche * | 1,5 | |
| A | US-A-3 671 523 (FARBENFABRIKEN BAYER AG) * Ansprüche * | 1,5 | |
| A | DE-A-2 726 016 (CIBA-GEIGY AG) * Ansprüche * | 1,5 | RECHERCHIERTE SACHGEBIETE (Int. Cl. ³) |
| P,Y | CHEMICAL ABSTRACTS, vol. 100, 1984, page 604, no. 209879w, Columbus, Ohio; & JP - A - 58 216 173 (SUMITOMO CHEMICAL CO., LTD.) 15.12.1983 * Zusammenfassung * | 1,5 | C 07 D 253/00 |

Der vorliegende Recherchenbericht wurde für alle Patentansprüche erstellt.

| Recherchenort | Abschlußdatum der Recherche | Prüfer |
|---|---|---|
| DEN HAAG | 28-09-1984 | VAN BIJLEN H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82